(19) Europäisches Patentamt
European Patent Office
Office européen des brevets

(11) Publication number: **0 304 322 B1**

(12) **EUROPEAN PATENT SPECIFICATION**

(45) Date of publication of patent specification: **02.12.92**

(51) Int. Cl.[5]: **C07D 241/44**, A61K 31/495

(21) Application number: **88307711.7**

(22) Date of filing: **19.08.88**

(54) **1-Substituted alkyl-2-oxo-1,2-dihydroquinoxaline derivatives.**

(30) Priority: **21.08.87 JP 207989/87**

(43) Date of publication of application:
**22.02.89 Bulletin 89/08**

(45) Publication of the grant of the patent:
**02.12.92 Bulletin 92/49**

(84) Designated Contracting States:
**CH DE ES FR GB IT LI**

(56) References cited:
**EP-A- 0 008 864**
**EP-A- 0 032 564**
**US-A- 4 262 123**

(73) Proprietor: **ASAHI KASEI KOGYO KABUSHIKI KAISHA**
**2-6, Dojimahama 1-chome Kita-ku**
**Osaka(JP)**

(72) Inventor: **Yaso, Masao**
**848-1, Takyo Ohito-cho**
**Tagata-gun Shizuoka-ken(JP)**
Inventor: **Suzuki, Yukio**
**530-1 Yokkamachi Nirayama-cho**
**Tagata-gun Sizuoka-ken(JP)**
Inventor: **Honda, Eiichi**
**11-27 Hon-cho**
**Mishima-shi Shizuoka-ken(JP)**
Inventor: **Shibata, Kensuke**
**846-3 Nanjo Nirayama-cho**
**Tagata-gun Shizuoka-ken(JP)**
Inventor: **Kinoshita, Hiroyuki**
**379-1 Mifuku Ohito-cho**
**Tagata-gun Shizuoka-ken(JP)**
Inventor: **Takayanagi, Noriyasu**
**284-4 Tokura Shimizu-cho**
**Sunto-gun Shizuoka-ken(JP)**
Inventor: **Saito, Tetsu**
**404-250 Kawaragaya**
**Mishima-shi Shizuoka-ken(JP)**
Inventor: **Hayashi, Eiichi**
**7-32 Midori-cho**
**Shizuoka-shi Shizuoka-ken(JP)**

(74) Representative: **Bentham, Stephen et al**
**J.A. Kemp & Co. 14 South Square Gray's Inn**
**London WC1R 5EU(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 304 322 B1

## Description

This invention relates to 1-substituted alkyl-2-oxo-1,2-dihydroquinoxaline derivatives having sympatholytic α-receptor blocking activity useful for the treatment of hypertension.

Known 1-substituted-2-oxo-3-substituted-1,2-dihydroquinoxaline derivatives having pharmacological activity are: 1-(amino, lower alkylamino or dilower alkylamino)-lower alkyl-2-oxo-3-lower alkyl-1,2-dihydroquinoxaline useful as a tranquilizer (Japan. Pat. Publ. No. 45-19907); 1-diethylaminoethyl-2-oxo-3-(benzyl or substituted benzyl)-1,2-dihydroxyquinoxaline having antispasmolytic or platelet aggregation activity (Japan. Pat. Publ. No. 46-11183, Japan. Pat. Unexam. Publs. No. 56-97226 and No. 56-97228); 1-alkyl-2-oxo-3-substituted carbamoyloxymethyl-1,2-dihydroquinoxaline effective against arteriosclerosis and thrombosis (Japan. Pat. Unexam. Publ. No. 49-24981); 1-alkyl-2-oxo-3-(tetrazole-5-yl carbamoyl)-1,2-dihydroquinoxaline useful for the treatment of extrinsic asthma and urticaria (Japan. Pat. Unexam. Publ. No/ 50-29583); 1-substituted phenyl-2-oxo-3-(alkyl, carboxyalkyl or dicarboxyalkyl)-1,2-dihydroquinoxaline is useful against inflammation and rheumatoid arthritis (Japan. Pat. Unexam. Publ. No. 55-115875); and 1-(phenyl or substituted phenyl)-2-oxo-3-(amino or alkaneamide)1,2-dihydroquinoxaline-4-oxide having an antispastic action, ataractic action of psycholeptic activity (Japan. Pat. Unexam. Publ. No. 56-92277).

Further quinoxaline derivatives which are useful for the treatment of hypertension are known, i.e. : 1-isopropylamino-3-(2-(2-quinoxalyl)phenyl-2-propanol (Japan. Pat. Publ. No. 43-9220), 2-methyl-3-(2-(4-phenyl or tryl)-(piperazinyl)ethoxyquinoxaline (Japan. Pat. Publ. No. 48-21949), 5-halogeno-6-(2-imidazolin-2-yl-amino)-quinoxaline (Japan. Pat. Unexam. Publ. No. 48-97878), 2-methyl-3-(2-hydroxyquinoxaline-6 or 7-yl) alanine (Japan. Pat. Unexam. Publ. No. 52-97933), 5- or 8-(2-hydroxy-3-substituted amino-propoxy)-2-oxo-3-methyl quinoxaline (Japan. Pat. Unexam. Publ. No. 55-162783) and 4-fluoro-N-{2-[4-(5-quinoxalyl)-1-piperazinyl]ethyl}benzamide (Japan. Pat. Unexam. Publ. No. 60-104063).

It is of great importance to find excellent pharmaceuticals having stronger inhibitory activities.

EP-A-0,032,564 discloses that the following compound (caroverin) has various pharmaceutical properties:

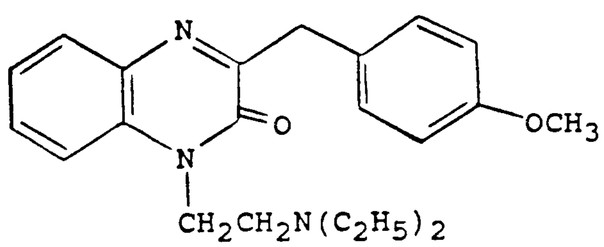

We have found certain 1-[1-(4-substituted piperazinyl)]alkyl-2-oxo-3-lower alkyl or non-substituted-1,2-dihydroquinoxalines having sympatholytic α-receptor blocking activity with pharmaceutically interesting properties.

The present invention provides a compound which is a 1-alkyl-2-oxo-1,2-dihydroquinoxaline derivative of formula [1]:

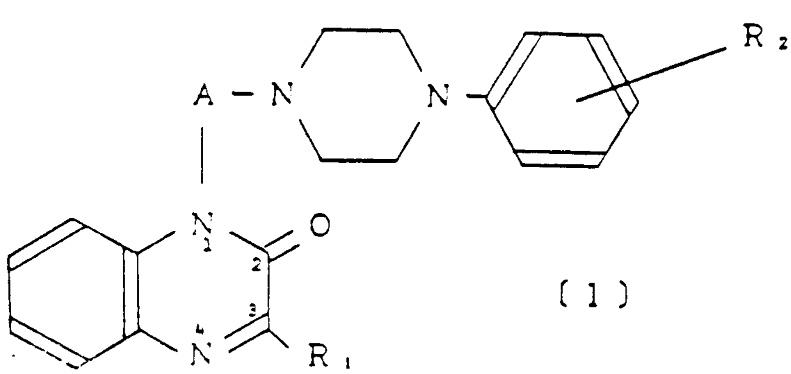

[1]

wherein $R_1$ is hydrogen or $C_{1-6}$ (preferably $C_{1-4}$) alkyl, $R_2$ is hydrogen, $C_{1-6}$ (preferably $C_{1-4}$) alkoxy or halogen, and A is $C_{1-6}$ (preferably $C_{1-4}$) alkylene, or a pharmacologically acceptable non-toxic salt thereof.

The compound of formula [1] can be provided in the form of a pharmacologically acceptable non-toxic salt. Examples of salts are salts of inorganic acids such as hydrochloride, sulfate or phosphate salts, and salts of organic acids such as acetate, propionate, glycolate, gluconate, malate, tartrate, succinate, mandelate, glutamate, aspartate, methanesulfonate and toluenesulfonate salts.

The compound of formula [1] can be produced by the following process.

A 2-oxo-1,2-dihydroquinoxaline derivative of formula [2]:

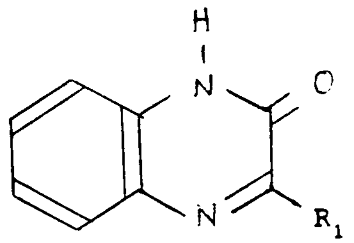

( 2 )

as defined above is reacted with a hydroxyalkyl halide of formula

$X_1$-A-OH

wherein $X_1$ is a halogen and A is as defined above in an organic solvent, to obtain a 1-hydroxyalkyl-2-oxo-1,2-di-hydroquinoxalinederivative of formula [3]

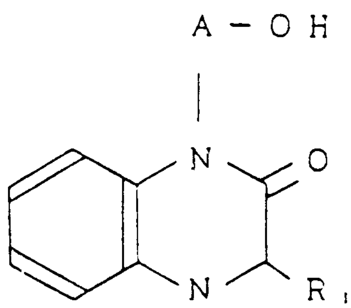

( 3 )

wherein $R_1$ and A have the same meanings hereinabove, then halogenating the said compound [3] with halogenating agent to obtain 1-halogenoalkyl-2-oxo-1,2-dihydroquinoxaline of the formula

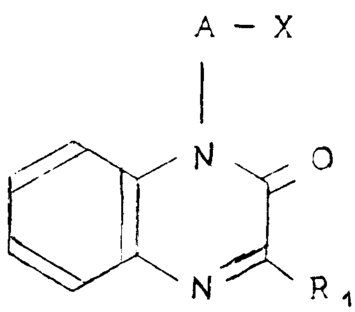

( 4 )

wherein X is halogen, and $R_1$ and A have the same meanings hereinabove, thereafter reacting the said compound [4] with an amine of the formula

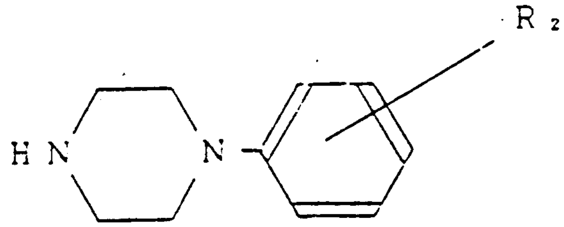

[5]

wherein $R_2$ has the same meaning hereinabove, in the presence of a base and in an organic solvent, and if desired converting the resultant compound of formula [1] into a pharmacologically acceptable non-toxic salt thereof.

In the compound (2) $R_1$ is hydrogen or $C_{1-6}$ alkyl, preferably $C_{1-4}$ alkyl, which may optionally be branched. Examples are methyl, ethyl, propyl, isopropyl, butyl, isobutyl, sec-butyl or t-butyl. The compounds (2) can be produced by the process disclosed in J. Am. Chem. Soc., 75: 328 (1953) and ibid. 76: 287 (1954).

The group A in the above hydroxyalkyl halide is $C_{1-6}$ alkylene, for example methylene, ethylene, methylmethylene, propylene, 1-methylethylene, dimethylethylene or 1-ethylmethylene. Ethylene is preferred.

The group X in the above hydroxyalkyl halide is a halogen such as chlorine or bromine. In general chlorine is preferred. The preferred compound is ethylene chlorohydrine.

The reaction of the compound (2) with the hydroxyalkyl halide proceeds in an organic solvent such as t-butanol.

The above reaction proceeds preferably in an aqueous alkali such as a hydroxy alkali, and preferably under heating. Isolation of the product (3) can be performed by adding water to the reaction mixture and extracting with a water immiscible organic solvent.

The above compound (4) can be produced by halogenating the compound (3) with a halogenating agent.

Examples of the halogenating agent are known halogenating agents such as $SOCl_2$, $PCl_5$ or $POCl_3$. The halogenation reaction can be carried out, in general, in an inert organic solvent such as chloroform. The reaction proceeds at room temperature. The product (4) can be isolated by adding a water immiscible organic solvent such as chloroform, washing with dilute aqueous alkali, dehydrating the organic layer and removing the solvent therefrom.

The compound (4) can be purified, if required, for example by silica-gel column chromatography.

The compound (1) can be produced by reacting the compound (4) with the amine (5).

The group $R_2$ in the amine (5) is hydrogen, $C_{1-6}$ alkoxy or halogen. Examples of $C_{1-6}$ alkoxy are $C_{1-4}$ alkoxy which may optionally be branched, such as methoxy, ethoxy, propoxy, isopropoxy, 1-methylethoxy, butoxy, isobutoxy, sec-butoxy or t-butoxy. An example of the halogen is chlorine.

Accordingly examples of the amine (5) are 4-phenylpiperazine, 4-(o-, m- or p-chlorophenyl)-piperazine or 4- (o-, m- or p-methoxyphenyl)-piperazine.

The reaction of the compound (4) and the amine (5) conventionally proceeds in an inert organic solvent such as benzene under heating. In the reaction hydrogen halogenide is generated so that the reaction preferably is carried out in the presence of an acid binder, for example a tertiary organic amine such as triethylamine. The compound (1) can be isolated by pouring the reaction mixture into dilute aqueous alkali and extracting with a water immiscible organic solvent such as benzene or chloroform.

The thus obtained compound (1) is purified, if required, by column chromatography using silica-gel, activated alumina or an adsorption resin with an elution solvent such as chloroform-methanol or benzene-ethyl acetate.

The compound (1) is in general produced in the form of a free base, but it can be produced in the form of a conventional salt thereof. For example, the hydrochloride can be prepared by dissolving the compound (1) in a $C_{1-6}$ alcohol such as methanol or ethanol, adding a calculated molar amount of hydrochloric acid, and isolating the precipitated material, or if not precipitated, by adding ether therein to precipitate.

Pharmacological actions of the compound (1) are illustrated below:

A test sample is prepared by dissolving a compound in the examples in a small amount of methanol, adding a two-molar amount of hydrochloric acid in methanol to precipitate the material, or if not precipitated, adding diethyl ether therein to precipitate, filtering the precipitate, and drying <u>in vacuo</u> to obtain

the dihydrochloride. The thus obtained test sample is dissolved in distilled water or physiological saline before use.

(1) α-blocking action:

A rat, Wistar, male, body weight 200 g, was sacrificed by bleeding. The extirpated spermatic duct was hung in Tyrode's solution at 37 ° C. The specimen duct was loaded at 0.5 g tension and nutrient solution was bubbled with a mixed gas of oxygen-carbon dioxide (95:5).

A constrictor, noradrenaline, 2 x $10^{-5}$ M was added in a nutrient solution to constrict the spermatic duct. At the maximum constriction, the spermatic duct was washed with nutrient solution, and this operation was repeated at 20 minute intervals until a constant constriction was observed. Thereafter a test compound, used after dissolving and diluting with distilled water, was added to the nutrient solution, and after three minutes noradrenaline was added to constrict the duct specimen. The reaction was recorded at an isotonic condition. A suppressive ratio (%) was calculated as the ratio of suppression on the constriction with the test compound to the constriction with adding noradrenaline. The results are shown in Table 1.

Furthermore the constrictor noradrenaline was replaced by barium chloride (2 x $10^{-3}$M), also shown in Table 1. As shown in the Table, since the test sample did not show the suppressive action against constriction for barium chloride at $10^{-6}$ g/ml, the above constriction suppressive action by noradrenaline is not based upon non-specific smooth muscle suppression, but is an action through the α-receptor.

Table 1

Test compound: 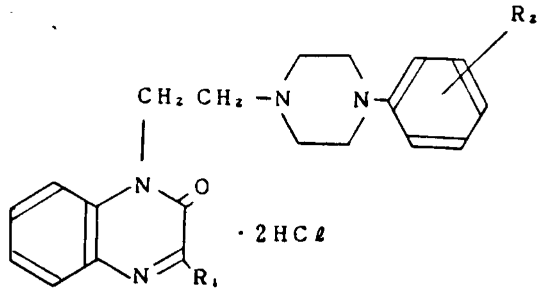 $\cdot 2HCl$

| Compound of the present invention | | | Suppressive ratio (%) | | | | | |
|---|---|---|---|---|---|---|---|---|
| No. of Example | $R_1$ | $R_2$ ( ): substituted position | noradrenaline | | | | | barium chloride |
| | | | concentration of test compound (g/ml) | | | | | |
| | | | $10^{-9}$ | $10^{-8}$ | $10^{-7}$ | $10^{-6}$ | $10^{-5}$ | $10^{-6}$ |
| 1 | H | $-OCH_3$ (o) | −3.1 | 30.8 | 96.0 | | | −3 |
| 2 | H | $-OCH_3$ (m) | | | 1.0 | 68.3 | 98.0 | |
| 3 | H | $-OCH_3$ (p) | | | −2.0 | 26.3 | 88.3 | |
| 4 | H | $-Cl$ (o) | −0.3 | 19.0 | 89.8 | | | −8 |
| 5 | H | $-Cl$ (m) | | 1.0 | 27.0 | 91.5 | | |

| | | | | | | | | |
|---|---|---|---|---|---|---|---|---|
| 6 | H | -Cl (p) | | | 24.8 | 81.8 | 97.3 | |
| 7 | $CH_3$ | $-OCH_3$ (o) | 0.8 | 32.8 | 98.8 | | | -6 |
| 8 | $CH_3$ | -Cl (p) | | -0.3 | 23.0 | 85.5 | | |
| 9 | $CH_3$ | H | | 6.8 | 43.5 | 99.8 | | |

(2) Hypotensive action:

A rat, Wistar, male, body weight 320-430 g, was anaesthetized with urethane (1.0 g/kg, i.p.). The blood pressure was measured through pressure-transducer by cannulation in the femoral artery.

The test compound, dissolved and diluted in physiological saline before use, was administered at 0.05

ml/100 g body weight through a cannula in the femoral artery.

The test compound obtained in Example 4 showed hypotensive action of approximately 30% at 10 μg/kg administration and approximately 40% at 100 μg/kg, which continued after 60 minutes administration.

The test compound obtained in Example 7 showed the same level of hypotensive activity as the compound in Example 4. The maximum hypotensive activity of the test compound in Example 7 is slightly stronger than that of Example 4. However prolongation of activity is less than that of Example 4 for the 100 μg/kg administered group.

The following Examples further illustrate the present invention.

## Referential Example 1

1-(2-hydroxyethyl)-2-oxo-1,2-dihydroquinoxaline:

2-hydroxyquinoxaline (7.31 g, 50 mM) and 5N-NaOH (50 ml) were added to t-butanol (150 ml). Ethylene chlorohydrin (20.13 g, 0.25 M) was added therein and stirred at 60 °C for 165 minutes. The organic solvent was removed in vacuo. Water was added to the residue and extracted with chloroform (once with 250 ml and twice with 50 ml). The combined chloroform layer was dried with anhydrous sodium sulfate and concentrated in vacuo. The residue was charged on a column of silica-gel (Wakogel C-200, Wako Pure Chem. Co.) and eluted with chloroformmethanol (50:1) to obtain 1-(2-hydroxyethyl)-2-oxo-1,2-dihydroquinoxaline (8.11 g, yield : 85.4%).

NMR ($\delta^{TMS}_{CDCl_3}$) ; 2.67 (1H、t、J=6)、3.8~4.2 (2H、m)、4.3~4.6 (2H、m)、7.2~8.0 (4H、m)、8.27 (1H、s) ppm

Mass (CI) ; 191 ($M^+$ + 1)、173

IR (Nujol) ; 1645 ; 1605 ; 1590 $cm^{-1}$

## Examples 1 - 6

1-[2-(4-substituted phenylpiperazinyl)ethyl]-2-oxo-1,2-dihydroquinoxaline:

Thionylchloride (0.42 ml) was added dropwise under ice-cooling into 1-(2-hydroxyethyl)-2-oxo-1,2-dihydroquinoxaline (0.95 g, 5 mM) dissolved in chloroform (10 ml), and stirred at room temperature for chlorination. Benzene (30 ml) and triethylamine were added to the reaction mixture, amine (10 mM) was further added and the mixture was refluxed. Dilute aqueous potassium carbonate was added to the reaction mixture and extracted with chloroform. The extract was dried with anhydrous sodium sulfate and concentrated in vacuo. The residue dissolved in a small amount of chloroform was charged on a column of silica-gel (C-200, 80g) and eluted with chloroform-methanol to obtain 1-[2-(4-substituted phenylpiperazinyl)-ethyl]-2-oxo-1,2-dihydroquinoxaline. Table 2 illustrates the chlorination time, type of amine, amount of amine used, amount of triethylamine used, reflux time, ratio of eluent and yields.

Table 2

Test compound : 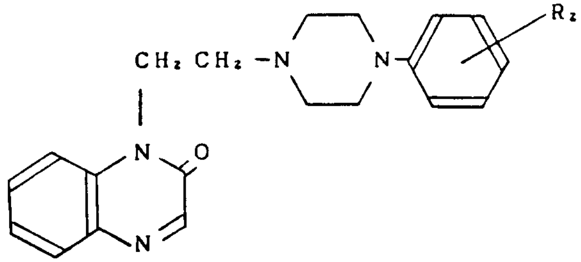

| Example | Chlorination time ( h ) | amine 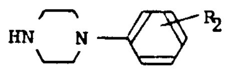 ( ): salt | amount used | trietylamine (ml) | veflux time ( h ) | ratio of eluent | Yield ( g ) | Yield ( % ) |
|---|---|---|---|---|---|---|---|---|
| 1 | 8 | o−OCH₃ | 1. 92 | 2. 8 | 14 | 500:1 | 0. 38 | 20. 0 |
| 2 | 14 | m−OCH₃ | 1. 92 | 2. 8 | 8 | 500:1 | 0. 38 | 20. 0 |
| 3 | 8 | p−OCH₃ | 1. 92 | 2. 8 | 14 | 500:1 | 0. 24 | 13. 2 |
| 4 | 14 | o−Cl (2HCl) | 2. 70 | 4. 2 | 8 | 1000:1 | 0. 45 | 24. 4 |
| 5 | 8 | m−Cl (2HCl) | 2. 70 | 4. 2 | 14 | 1000:1 | 0. 47 | 25. 5 |
| 6 | 14 | p−Cl (2HCl) | 2. 70 | 14 | 8 | 1000:1 | 0. 08 | 4. 3 |

NMR ( $\delta^{TMS}_{CDCl_3}$ ) and Mass (CI) spectra are:

Compound of Example 1

NMR ；2.6～2.9（6H、m）、3.9～3.2（4H、m）、3.86（3H、s）、4.45（2H、t、J＝8）、6.7～8.0（8H、m）、8.30（1H、s）ppm

Mass ；365、205

Compound of Example 2

NMR ；2.6～2.9（6H、m）、3.0～3.3（4H、m）、3.79（3H、s）、4.44（2H、t、J＝8）、6.5～6.6（3H、m）、7.00～8.0（5H、m）、8.30（1H、s）ppm

Mass ；365、205

Compound of Example 3

NMR ；2.6～2.9（6H、m）、2.9～3.2（4H、m）、3.77（3H、s）、4.44（2H、t、J＝8）、6.7～7.0（4H、m）、7.2～8.0（4H、m）、8.30（1H、s）ppm

Mass ；365、205

Compound of Example 4

NMR ；2.6～2.9（6H、m）、2.9～3.2（4H、m）、4.45（2H、t、J＝8）、6.8～8.0（8H、m）、8.31（1H、s）ppm

Mass ；371、369、211、209

Compound of Example 5

NMR ; 2.6~2.9 (6H、m)、3.0~3.3 (4H、m)、4.44 (2H、t、J=8)、6.6~8.0 (8H、m)、8.30 (1H、s) ppm

Mass ; 371、369、211、209

Compound of Example 6

NMR ; 2.6~2.9 (6H、m)、3.0~3.3 (4H、m)、4.44 (2H、t、J=8)、6.7~8.0 (8H、m)、8.30 (1H、s) ppm

Mass ; 371、369、211、209

Referential Example 2

1-(2-hydroxyethyl)-2-oxo-3-methyl-1,2-dihydroquinoxaline:

3-Methyl-2-hydroxyquinoxaline (4.8g, 30 mM) was added to t-butanol (90 ml). 5N NaOH (30 ml) was added therein, heated at 60°C. Ethylene chlorohydrin (10.2 ml, 150 mM) was added therein and stirred at 60°C for 3-4 hours. The Precipitated by-product was removed by filtration and the filtered solution was concentrated in vacuo. The residue was extracted with chloroform (100 ml) and water (100 ml). The aqueous layer was extracted several times with chloroform (100 ml). The combined chloroform layer was dried with anhydrous sodium sulfate and concentrated in vacuo. Acetone (150 ml) was added to the residue, heated, and cooled for recrystallization to obtain 1-(2-hydroxyethyl)-2-oxo-3-methyl-1,2-dihydroquinoxaline as needle crystals. Yield: 5.19 g (yield: 85.8%).

NMR ($\delta^{TMS}_{CDCl_3}$) ; 2.57 (3H、s)、4.05 (2H、t、J=5.5)、4.50 (2H、t、J=5.5)、7.2~7.86 (4H、m) ppm

IR (Nujol) ; 1645 ; 1610 (amino) $cm^{-1}$

Examples 7 - 9

1-[2-(4-substituted phenylpiperazinyl)ethyl]-2-oxo-3-methyl-1,2-dihydroquinoxaline:

Thionylchloride (0.44 ml. 6mM) was added under ice-cooling to 1-(2-hydroxyethyl)-2-oxo-3-methyl-1,2-dihydroquinoxaline (1.02 g, 5mM) dissolved in chloroform (15 ml), and stirred at room temperature for 4-6 hours. Benzene (20 ml) and triethylamine (4.18 ml, 30 mM) were added to the reaction mixture, amine (10 mM) was further added and the mixture was refluxed for 24 hours. The reaction mixture was concentrated

in vacuo and chloroform (60 ml) and water (100 ml) were added to the residue, then extracted with chloroform. The water layer was extracted 2-3 times with chloroform (60 ml). The combined extract was dried with anhydrous sodium sulfate and concentrated in vacuo. The residue, dissolved in a small amount of chloroform, was charged on a column of silica-gel (C-200, 160 g) and eluted with chloroform-methanol (200:1) to obtain 1-[2-(4-substituted phenylpiperazinyl)ethyl]-2-oxo-1,2-dihydroquinoxaline.
Table 3 illustrates the type of amine, the amount used and yields.

Table 3

Compound:

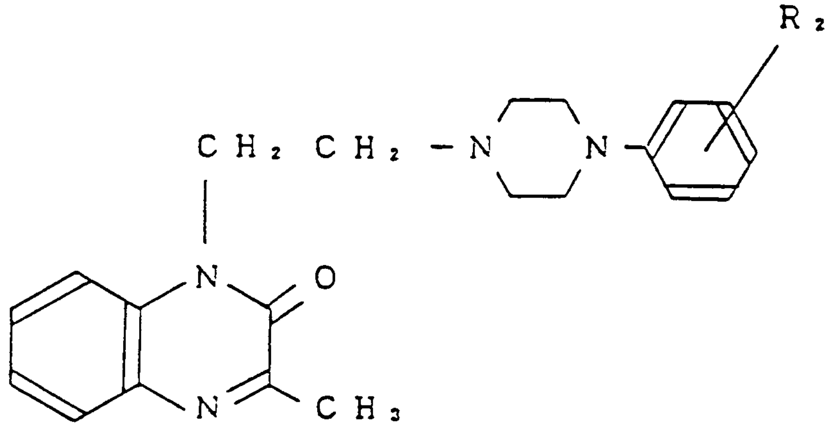

amine ;

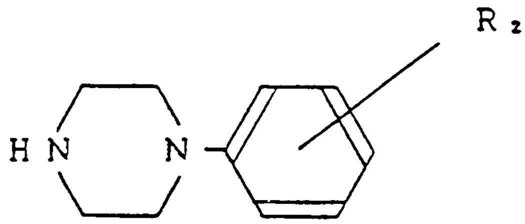

| Example | Amine | | yield (%) | yield (%) |
|---|---|---|---|---|
| | $R_2$ ( ): salt | amount used (g) | | |
| 7 | o-$OCH_3$ | 1.92 | 1.02 | 27.0 |
| 8 | p-Cℓ (2HCℓ) | 2.70 | 0.22 | 5.8 |
| 9 | H | 1.53 | 0.73 | 42.0 |

NMR ($\delta^{TMS}_{CDCl_3}$) and Mass (CI) spectra are:

Compound of Example 7

NMR ; 2.60 (3H, s), 2.77 (2H, t, J=7.6), 2.81 (4H, t, J=3.7), 3.13 (4H, t, J=3.7), 3.86 (3H, s), 4.45 (2H, t, J=7.6), 6.81~7.87 (8H, m) ppm

Mass ; 379 ($M^+$+1), 219, 205, 187

Compound of Example 8

NMR ; 2.60 (3H、s)、2.75 (2H、t、J=7.0)、2.75 (4H、t、J=5.3)、3.18 (4H、t、J=5.3)、4.45 (2H、t、J=7)、6.77～7.86 (8H、m) ppm

Mass ; 385、383、223、209、187

Compound of Example 9

NMR ; 2.60 (3H、s)、2.77 (2H、t、J=7.5)、2.74 (4H、t、J=5.3)、3.23 (4H、t、J=5.3)、4.46 (2H、t、J=7.5)、6.85～7.86 (9H、m) ppm

Mass ; 349 ($M^+$ +1)、188

## Claims

### Claims for the following Contracting States : CH, DE, FR, GB, IT, LI

**1.** A compound which is a 1-alkyl-2-oxo-1,2-dihydroquinoxaline derivative of formula [1]:

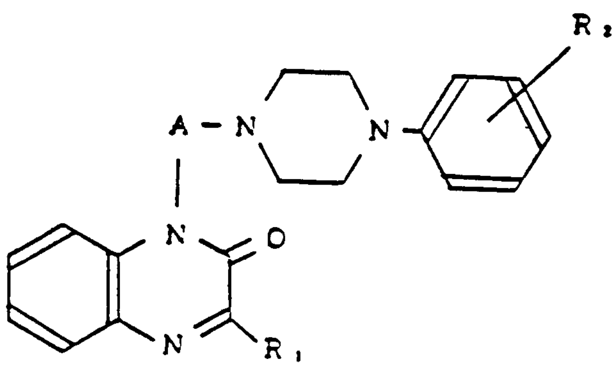

[1]

wherein $R_1$ is hydrogen or $C_{1-6}$ alkyl, $R_2$ is hydrogen, $C_{1-6}$ alkoxy or halogen, and A is $C_{1-6}$ alkylene, or a pharmacologically acceptable non-toxic salt thereof.

**2.** A compound according to Claim 1 wherein $R_1$ is methyl and A is ethylene.

**3.** A compound according to Claim 1 or 2 wherein $R_2$ is hydrogen, methoxy or chlorine.

**4.** A process for preparing a compound as defined in Claim 1 which comprises reacting a compound of formula [4]:

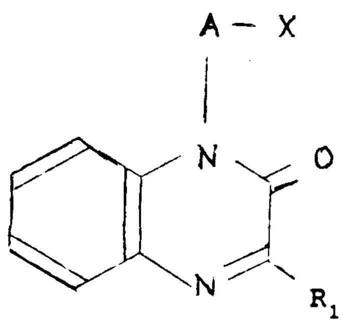

(4)

wherein X is a halogen and $R_1$ and A are as defined in Claim 1 with an amine of formula [5]:

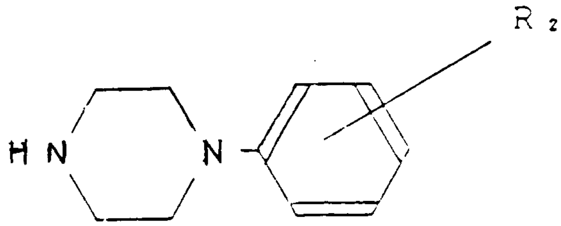

(5)

wherein $R_2$ is as defined in Claim 1, in the presence of a base and in an organic solvent, and if desired converting the resultant compound of formula [1] into a pharmacologically acceptable non-toxic salt thereof.

**5.** A process according to Claim 4 wherein the compound of formula [4] is prepared by halogenating a compound of formula [3]:

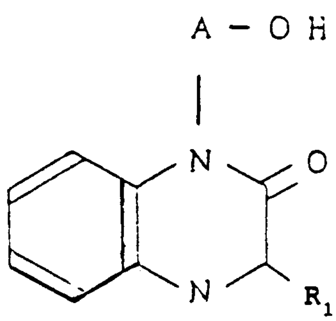

(3)

wherein $R_1$ and A are as defined in Claim 4 with a halogenating agent.

**6.** A process according to Claim 5 wherein the compound of formula [3] is prepared by reacting a compound of formula [2]:

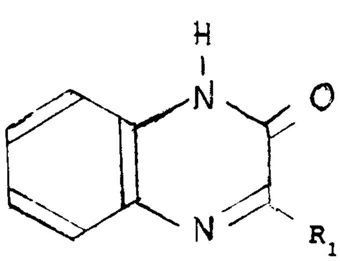

(2)

wherein $R_1$ is as defined in Claim 5 with a hydroxyalkyl halide of formula:

$X_1$-A-OH

wherein $X_1$ is a halogen and A is as defined above in an organic solvent.

7. A pharmaceutical composition comprising a compound as defined in claim 1 and a pharmaceutically acceptable carrier or diluent.

8. A compound as defined in Claim 1 for use in a method of treatment of the human or animal body by therapy.

9. A compound as defined in Claim 1 for use in a method of treatment of hypertension.

10. Use of a compound as defined in Claim 1 in the manufacture of a medicament for the treatment of hypertension.

**Claims for the following Contracting States : ES**

1. A process for preparing a compound which is a 1-alkyl-2-oxo-1,2-dihydroquinoxaline derivative of formula [1]:

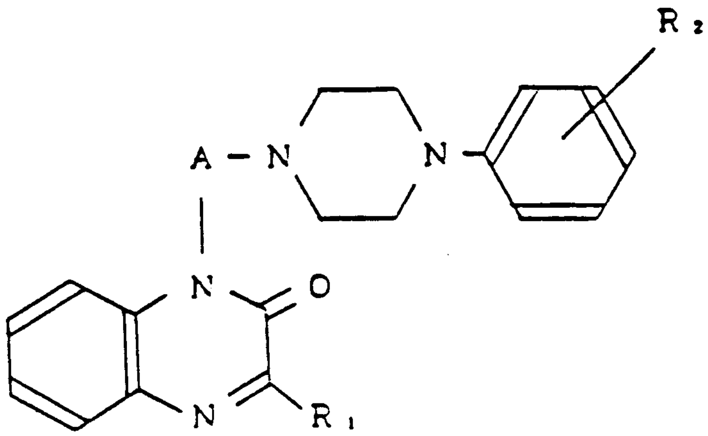

[1]

wherein $R_1$ is hydrogen or $C_{1-6}$ alkyl, $R_2$ is hydrogen, $C_{1-6}$ alkoxy or halogen, and A is $C_{1-6}$ alkylene, or a pharmacologically acceptable non-toxic salt thereof which comprises reacting a compound of formula [4]:

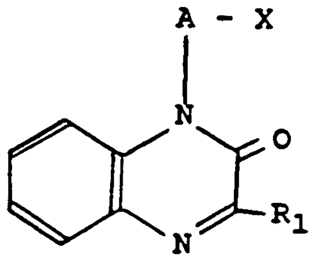

[4]

wherein X is a halogen and $R_1$ and A are as defined above with an amine of formula [5]:

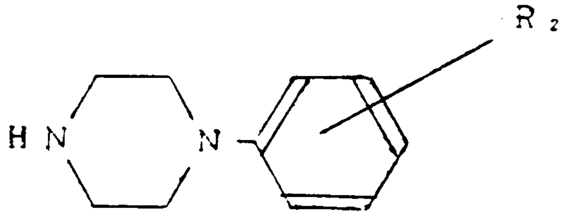

( 5 )

wherein $R_2$ is as defined above, in the presence of a base and in an organic solvent, and if desired converting the resultant compound of formula [1] into a pharmacologically acceptable non-toxic salt thereof.

**2.** A process according to Claim 1 wherein the compound of formula [4] is prepared by halogenating a compound of formula [3]:

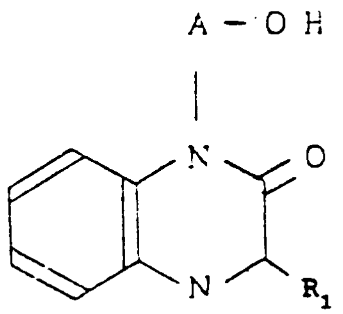

( 3 )

wherein $R_1$ and A are as defined in Claim 1 with a halogenating agent.

**3.** A process according to Claim 2 wherein the compound of formula [3] is prepared by reacting a compound of formula [2]:

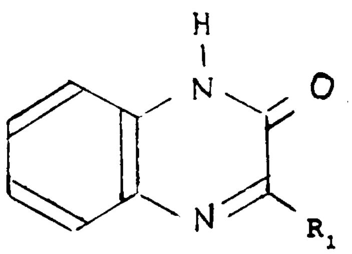

( 2 )

wherein $R_1$ is as defined in Claim 2 with a hydroxyalkyl halide of formula:

$X_1$-A-OH

wherein $X_1$ is a halogen and A is as defined above in an organic solvent.

**4.** A process according to any one of Claims 1 to 3 which further comprises mixing the compound of formula [1] or a pharmacologically acceptable non-toxic salt thereof with a pharmaceutically acceptable carrier or diluent.

**Patentansprüche**

**Patentansprüche für folgende Vertragsstaaten : CH, DE, FR, GB, IT, LI**

1. Verbindung in Gestalt eines 1-Alkyl-2-oxo-1,2-dihydrochinoxalin-Derivats der Formel [1]:

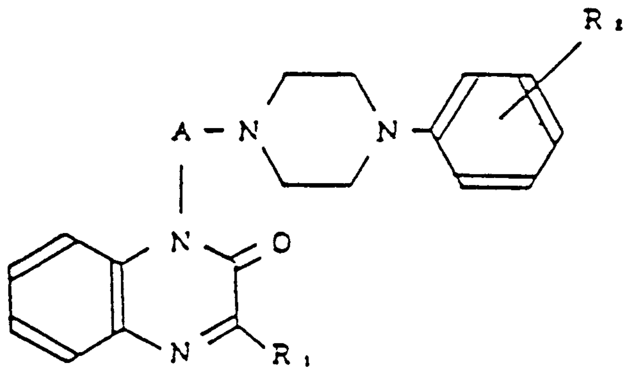

[1],

wobei $R_1$ die Bedeutung Wasserstoff oder $C_{1-6}$-Alkyl hat, $R_2$ die Bedeutung Wasserstoff, $C_{1-6}$-Alkoxy oder Halogen hat und A die Bedeutung $C_{1-6}$-Alkylen hat, oder ein pharmakologisch verträgliches nichttoxisches Salz davon.

2. Verbindung nach Anspruch 1, wobei $R_1$ die Bedeutung Methyl und A die Bedeutung Ethylen hat.

3. Verbindung nach Anspruch 1 oder 2, wobei $R_2$ die Bedeutung Wasserstoff, Methoxy oder Chlor hat.

4. Verfahren zur Herstellung einer Verbindung gemäß Definition in Anspruch 1, bei dem man eine Verbindung der Formel [4]

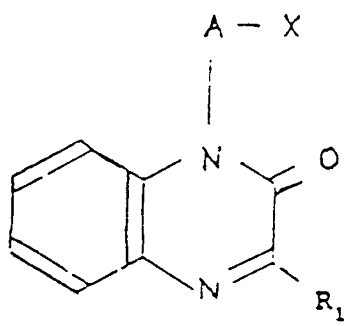

[4],

wobei X ein Halogen bedeutet und $R_1$ und A die in Anspruch 1 definierte Bedeutung haben, mit einem Amin der Formel [5]

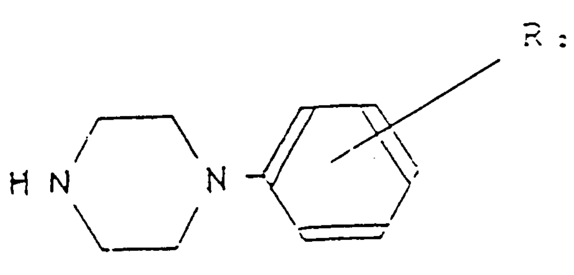

[5],

wobei $R_2$ die in Anspruch 1 definierte Bedeutung hat, in Gegenwart einer Base und in einem organischen Lösemittel umsetzt und gewünschtenfalls die entstehende Verbindung der Formel [1] in ein pharmakologisch verträgliches nichttoxisches Salz davon überführt.

5. Verfahren nach Anspruch 4, wobei man die Verbindung der Formel [4] herstellt durch Halogenieren einer Verbindung der Formel [3]

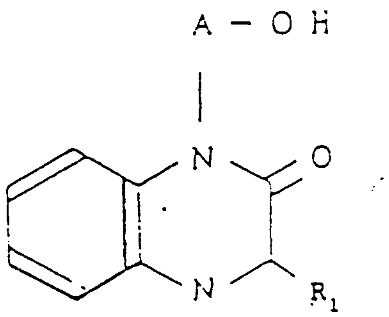

[3]

mit einem Halogenierungsmittel, wobei $R_1$ und A die in Anspruch 4 definierte Bedeutung haben.

6. Verfahren nach Anspruch 5, wobei man die Verbindung der Formel [3] herstellt durch Umsetzen einer Verbindung der Formel [2]

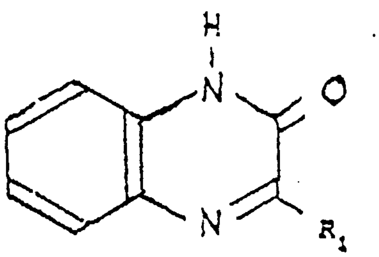

[2]

mit einem Hydroxyalkylhalogenid der Formel

$X_1$-A-OH

in einem organischen Lösemittel, wobei $R_1$ die in Anspruch 5 definierte Bedeutung hat und wobei $X_1$ ein Halogen bedeutet und A die vorstehend definierete Bedeutung hat.

7. Pharmazeutische Zusammensetzung enthaltend eine Verbindung gemäß Definition in Anspruch 1 und einen pharmazeutisch verträglichen Trägerstoff oder Verdünnungsstoff.

8. Verbindung gemäß Definition in Anspruch 1 zur Verwendung in einem Verfahren zur Behandlung des menschlichen oder tierischen Körpers durch Therapie.

9. Verbindung gemäß Definition in Anspruch 1 zur Verwendung in einem Verfahren zur Behandlung von Hypertension.

10. Verwendung einer Verbindung gemäß Definition in Anspruch 1 zum Herstellen eines Medikaments zur Behandlung von Hypertension.

**Patentansprüche für folgenden Vertragsstaat : ES**

1. Verfahren zum Herstellen einer Verbindung in Gestalt eines 1-Alkyl-2-oxo-1,2-dihydrochinoxalin-Derivats der Formel [1]:

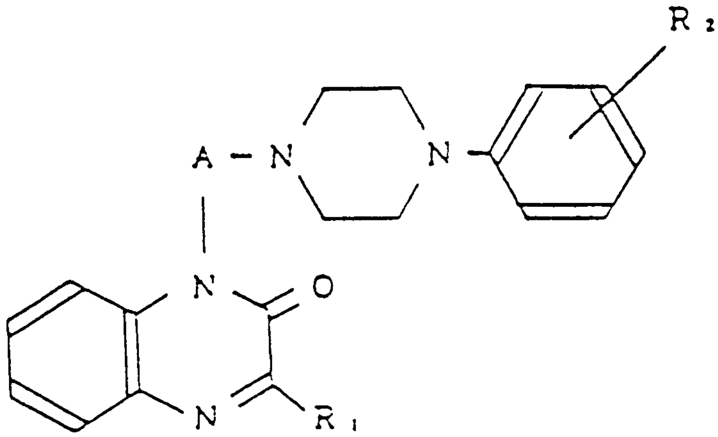

[1],

wobei $R_1$ die Bedeutung Wasserstoff oder $C_{1-6}$-Alkyl hat, $R_2$ die Bedeutung Wasserstoff, $C_{1-6}$-Alkoxy oder Halogen hat und A die Bedeutung $C_{1-6}$-Alkylen hat, oder eines pharmakologisch verträglichen nichttoxischen Salzes davon, bei dem man eine Verbindung der Formel [4]

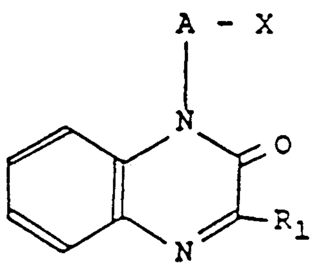

[4],

wobei X ein Halogen bedeutet und $R_1$ und A die vorstehend definierte Bedeutung haben, mit einem Amin der Formel [5]

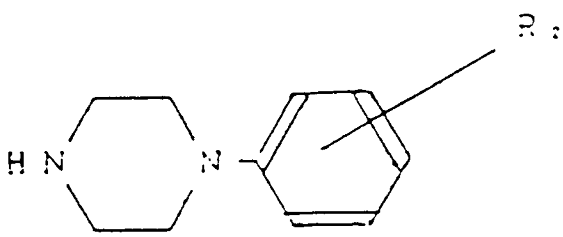

[5],

wobei $R_2$ die in Anspruch 1 definierte Bedeutung hat, in Gegenwart einer Base und in einem organischen Lösemittel umsetzt und gewünschtenfalls die entstehende Verbindung der Formel [1] in ein pharmakologisch verträgliches nichttoxisches Salz davon überführt.

**2.** Verfahren nach Anspruch 1, wobei man die Verbindung der Formel [4] herstellt durch Halogenieren einer Verbindung der Formel [3]

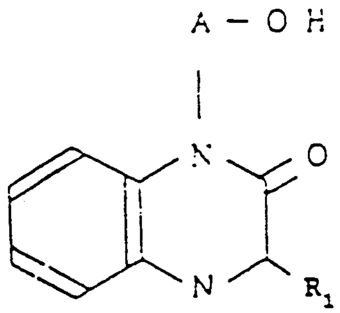

[3]

mit einem Halogenierungsmittel, wobei $R_1$ und A die in Anspruch 1 definierte Bedeutung haben.

**3.** Verfahren nach Anspruch 2, wobei man die Verbindung der Formel [3] herstellt durch Umsetzen einer Verbindung der Formel [2]

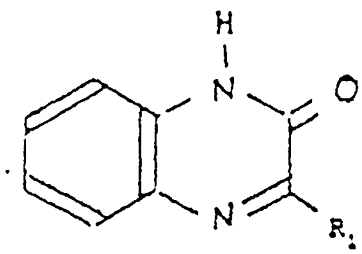

[2]

mit einem Hydroxyalkylhalogenid der Formel

$X_1$-A-OH

in einem organischen Lösemittel, wobei $R_1$ die in Anspruch 2 definierte Bedeutung hat und wobei $X_1$ ein Halogen bedeutet und A die vorstehend definierte Bedeutung hat.

**4.** Verfahren nach einem der Ansprüche 1 bis 3, wobei man weiter die Verbindung der Formel [1] oder ein pharmakologisch verträgliches nichtoxisches Salz davon mit einem pharmazeutisch verträglichen Träger oder Verdünnungsstoff mischt.

**Revendications**
**Revendications pour les Etats contractants suivants : CH, DE, FR, GB, IT, LI**

**1.** Composé qui est un dérivé de 1-alcoyl-2-oxo-1,2-dihydroquinoxaline de la formule (1) :

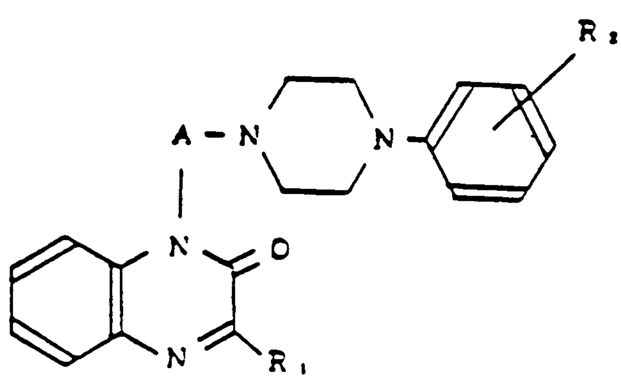

[1]

dans laquelle $R_1$ est un atome d'hydrogène ou un alcoyle à 1 à 6 atomes de carbone, $R_2$ est un atome d'hydrogène, un alcoxy à 1 à 6 atomes de carbone ou un atome d'halogène, et A est un alcoylène à 1 à 6 atomes de carbone, ou un sel non toxique pharmacologiquement acceptable de ce dérivé.

**2.** Composé selon la revendication 1, dans lequel $R_1$ est un groupe méthyle et A est un groupe éthylène.

**3.** Composé selon la revendication 1 ou la revendication 2, dans lequel $R_2$ est un atome d'hydrogène, un groupe méthoxy ou un atome de chlore.

**4.** Procédé pour préparer un composé comme défini dans la revendication 1, dans lequel on fait réagir un composé de la formule (4) :

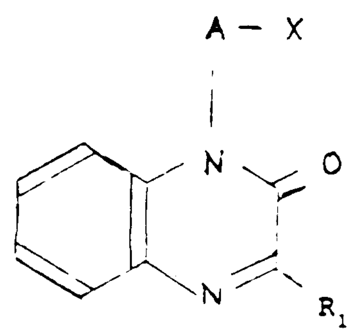

(4)

dans laquelle X est un atome d'halogène et $R_1$ et A sont comme définis dans la revendication 1, avec une amine de la formule (5) :

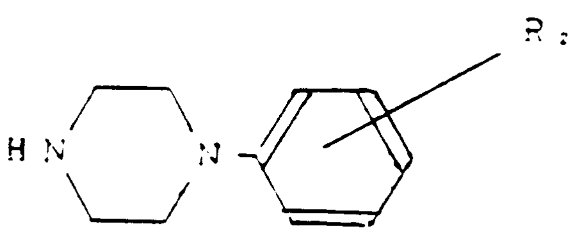

(5)

dans laquelle $R_2$ est comme défini dans la revendication 1, en la présence d'une base et dans un solvant organique, et, si on le désire, on convertit le composé résultant de la formule (1) en l'un de ses sels non toxiques pharmacologiquement acceptables.

**5.** Procédé selon la revendication 4, dans lequel le composé de la formule (4) est préparé en halogénant un composé de la formule (3) :

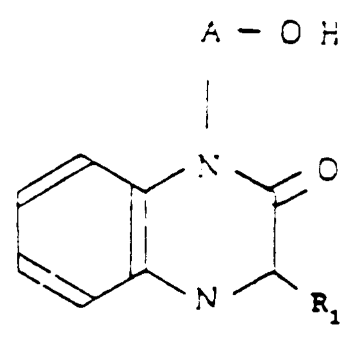

(3)

dans laquelle $R_1$ et A sont comme définis dans la revendication 4, avec un agent d'halogénation.

**6.** Procédé selon la revendication 5, dans lequel le composé de la formule (3) est préparé en faisant réagir un composé de la formule (2) :

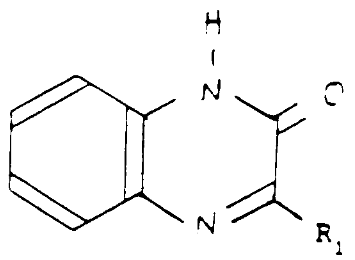

( 2 )

dans laquelle $R_1$ est comme défini dans la revendication 5 avec un halogénure d'hydroxyalcoyle de la formule :

$X_1$-A-OH

dans laquelle $X_1$ est un halogène et A est comme défini ci-dessus, dans un solvant organique.

**7.** Composition pharmaceutique contenant un composé selon la revendication 1 et un véhicule ou diluant pharmaceutiquement acceptable.

**8.** Composé selon la revendication 1, destiné à être utilisé pour le traitement du corps de personnes ou d'animaux par thérapie.

**9.** Composé selon la revendication 1, destiné à être utilisé pour le traitement de l'hypertension.

**10.** Utilisation d'un composé comme défini dans la revendication 1 pour la fabrication d'un médicament pour le traitement de l'hypertension.

**Revendications pour l'Etat contractant suivant : ES**

**1.** Procédé pour préparer un composé qui est un dérivé de 1-alcoyl-2-oxo-1,2-dihydroquinoxaline de la formule (1) :

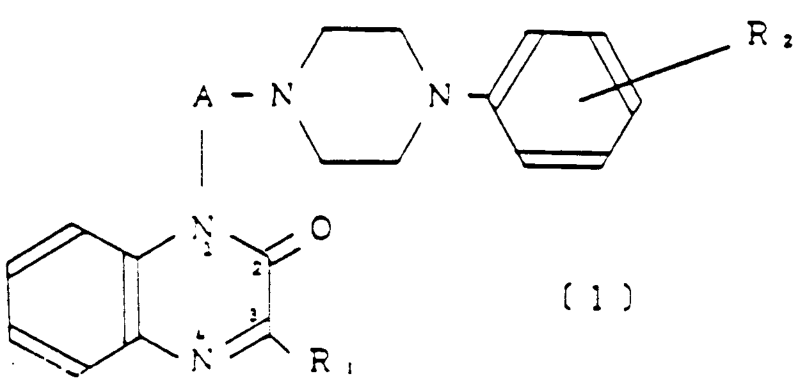

( 1 )

dans laquelle $R_1$ est un atome d'hydrogène ou un alcoyle à 1 à 6 atomes de carbone, $R_2$ est un atome d'hydrogène, un alcoxy à 1 à 6 atomes de carbone ou un atome d'halogène, et A est un alcoylène à 1 à 6 atomes de carbone, ou un sel non toxique pharmacologiquement acceptable de ce dérivé, dans lequel on fait réagir un composé de la formule (4) :

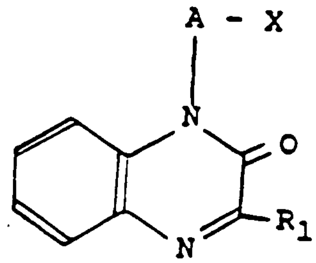

[4]

dans laquelle X est un atome d'halogène et $R_1$ et A sont comme définis ci-dessus avec une amine de la formule (5) :

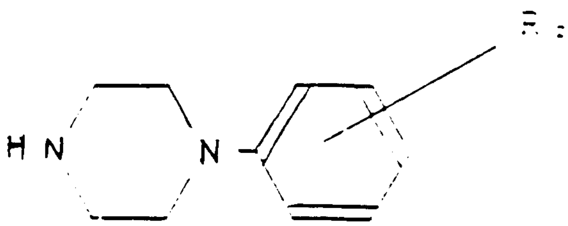

(5)

dans laquelle $R_2$ est comme défini ci-dessus, en la présence d'une base et d'un solvant organique et, si on le désire, on convertit le composé résultant de la formule (1) en un de ses sels non toxiques pharmacologiquement acceptables.

2. Procédé selon la revendication 1, dans lequel le composé de la formule (4) est préparé en halogénant un composé de la formule (3) :

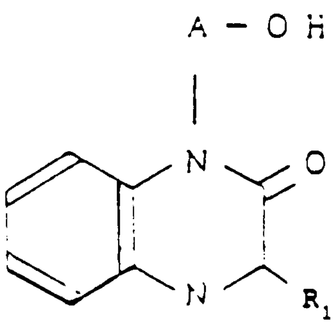

(3)

dans laquelle $R_1$ et A sont comme définis dans la revendication 1 avec un agent d'halogénation.

3. Procédé selon la revendication 2, dans lequel le compose de la formule (3) est préparé en faisant réagir un composé de la formule (2) :

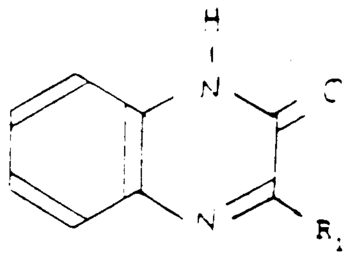

(2)

dans laquelle $R_1$ est comme défini dans la revendication 2, avec un halogénure d'hydroxyalcoyle de la formule :

$X_1$-A-OH

dans laquelle $X_1$ est un halogène et A est comme défini ci-dessus, dans un solvant organique.

4. Procédé selon l'une des revendications 1 à 3, dans lequel on mélange en outre le composé de la formule (1) ou un de ses sels non toxiques pharmacologiquement acceptables avec un véhicule ou un diluant pharmaceutiquement acceptable.